Europäisches Patentamt

European Patent Office

Office européen des brevets

(11) Publication number: **0 058 486**
**B1**

(12) **EUROPEAN PATENT SPECIFICATION**

(45) Date of publication of patent specification: **14.08.85**

(21) Application number: **82300457.7**

(22) Date of filing: **28.01.82**

(51) Int. Cl.⁴: **G 01 N 33/537,**
**G 01 N 33/548, G 01 N 33/78**

(54) Reusable assay for multisite antigens.

*ι'*

(30) Priority: **13.02.81 US 234414**

(43) Date of publication of application:
**25.08.82 Bulletin 82/34**

(45) Publication of the grant of the patent:
**14.08.85 Bulletin 85/33**

(84) Designated Contracting States:
**BE DE FR GB IT SE**

(56) References cited:
**DE-A-2 815 510**
**US-A-4 009 005**
**US-A-4 098 876**
**US-A-4 108 976**

**ANGEWANDTE CHEMIE, Vol. 88, No. 17, 1976,**
**Weinheim H.G. ECKERT "Die Technik des**
**Radioimmunoassays" pages 565 to 573**

(73) Proprietor: **Becton, Dickinson and Company**
**Mack Centre Drive**
**Paramus New Jersey 07652 (US)**

(72) Inventor: **Ferro, Ari M.**
**503, First Avenue No.4**
**Salt Lake City Utah 84103 (US)**
Inventor: **Smith, Richard S.**
**4607, S. Fortuna Way**
**Salt Lake City Utah (US)**
Inventor: **Krauth, Gary H.**
**3836 W. 9290 So.**
**W. Jordan Utah (US)**

(74) Representative: **Ruffles, Graham Keith et al**
**MARKS & CLERK 57-60 Lincoln's Inn Fields**
**London WC2A 3LS (GB)**

Courier Press, Leamington Spa, England.

## Description

This invention relates to an assay for ligands, and more particularly to an assay for antigens having multiple binding sites.

An assay for antigens having multiple binding sites is known in the art; eg, the so-called two-site IRMA (immunoradiometric assay), which involve insolubilization of the antigen by reaction with solid-phase antibody, followed by contact with radiolabelled antibody to provide both labelled antibody complexed with the antigen, and remaining free or uncomplexed labelled antibody. The amount of labelled material which has been complexed is a measure of the amount of antigen which was present. The so-called two-site IRMA has been employed for the assay of hTSH.

US Patent 4098876 discloses a method of determining the presence and/or concentration of a polyvalent antigenic substance in a fluid. The method comprises the steps of incubating the fluid with labelled antibodies to the substance to form a first labelled immunochemical complex and then incubating that complex with immobilized antibodies to the substance to form a second labelled complex which is separated from the incubation medium. The amount of label in the second complex provides a means for detecting and/or quantitating the substance in the fluid. By reversing the sequence of incubation steps in a "two-site" or sandwich assay, greater sensitivity is achieved and an intermediate washing step is eliminated.

The present invention is directed to an improvement in an assay for multisite antigens, and has particular applicability to an assay for hTSH.

In accordance with one aspect of the present invention, there is provided a process for the assay of an antigen having multiple binding sites, the assay comprising: contacting a sample containing said antigen with a labeled binder for said antigen to bind a portion of the labeled binder to the antigen and thereby give a mixture containing free labeled binder and a complex of labeled binder bound to said antigen; binding the said complex to a supported binder for the complex and thereby separating the bound labeled binder from the free labeled binder; and determining the amount of at least one of the bound and the free labeled binder; characterized in that the process is carried out using a flow-through chamber containing the supported binder for the complex, whereby the mixture is flowed through the chamber to bind the complex to the supported binder and an eluting liquid is then flowed through the chamber to elute the complex from the supported binder.

In accordance with a particularly preferred aspect of the invention, the assay is directed to an assay for hTSH.

The antigens which are assayed in accordance with the present invention are those which have multiple binding sites, with such antigen generally being characterized by having a higher molecular weight. Thus, for example such antigens are generally either polypeptides; in particular, hormones, or polypeptide proteins. As representative examples of such antigens, there may be mentioned hTSH (human thyroid stimulating hormones); HCG; insulin; CEA, ferritin, hepatitis associated antigens A and B, alpha-fetoprotein, growth hormone.

The binder employed as both the labelled binder and the supported binder is generally an antibody, and in some cases, may be a naturally occurring binder which can be isolated in a form specific for the antigen to be assayed. As known in the art, such antibodies are produced by introducing the antigen into a vertebrate.

The binder employed as the labeled binder, and the binder supported on the solid support for separating the labeled binder-antigen complex, as hereinabove noted, are both preferably antibodies. Such antibodies may be raised in the same species of animals; for example, rabbit; however, it is preferred to have the respective antibodies used as the labeled antibody, and as the supported antibody raised in different animals of such species to thereby ensure binding of different sites on the antigen by the labeled antibody and supported antibody, respectively.

The labeled antibody is preferably labeled with a suitable radioisotope, as known in the art. As representative examples of such radioisotopes, there may be mentioned radioisotopes of iodine, tritium, cobalt, with radio-iodine; and in particular $^{125}I$, being preferred. It is to be understood, however, that the use of other radioisotopes is within the scope of the invention. Similarly, it is also possible to label the antibody with other than a radioisotope; e.g., enzyme labeling or fluorescent labeling.

In regard to the supported antibody, such antibody may be supported on any one of a wide variety of solid materials. As known in the art, such materials include suitable polymers, such as polystyrene, polyethylene, polypropylene, polytetrafluoroethylene, polyamides, polyacrylamides, crosslinked agarose, dextran, glass, bacterial cells; ion exchange resins; cotton, as described in U.S. Patent No. 4,200,625 and supports of the type described in U.S. Patent No. 4,059,685. The selection of a suitable support is deemed to be within the scope of those skilled in the art from the teachings herein.

As known in the art, such antibody may be supported on the solid support by either adsorption, or by covalent coupling.

In performing the assay, the sample containing or suspected of containing the antigen is incubated with the labeled antibody, with such labeled antibody being employed in an amount which is in excess of the amount required to saturate all of the antigen binding sites for such labeled antibody; however, not-withstanding such excess, the labeled antibody does not saturate all of the binding sites of the antigen. The incubation is generally effected at temperatures in the order of from 15 to 40°C, and for a time sufficient to accomplish the binding. The resulting mixture will contain free labeled antibody, and labeled antibody bound to the antigen, as an immune complex.

After the incubation, the mixture is contacted with the supported antibody, to accomplish binding of the antigen-labeled antibody complex to the supported antibody through the remaining antigen binding sites. The free or uncoupled labeled antibody is not bound by the supported antibody, and as a result, there is a separation of the free labeled antibody from the labeled antibody bound to the antigen.

The amount of bound or complexed labeled antibody increases in proportion to the amount of antigen present in the sample, and as a result, the amount of antigen present in the sample can be determined by determining the amount of bound and/or free labeled antibody, and comparing same to a standard curve, by procedures known in the art.

In accordance with the invention, the complex of antigen labeled antibody, bound to the supported antibody, is eluted therefrom to thereby permit reuse of the supported antibody. In this respect, such elution is accomplished by the use of a suitable eluting liquid, which elutes the complex from the supported antibody, without destroying the binding ability of such supported antibody.

In accordance with a preferred embodiment, the eluting liquid is one which is at an acidic pH, which is no greater than 3.0, and at which the binding ability of the binder is not destroyed; i.e., the eluting liquid is not made too acidic. In general, the eluting liquid is at a pH of no less than 1.5, and preferably no less than 2.0.

In particular, such eluting liquid is water buffered to a pH as hereinbefore described.

The acidic pH may be obtained by the use of any one of a wide variety of acidic buffers. As representative examples of suitable buffers, there may be mentioned: citrates, acetates, glycinates, glutamates, oxalates, tartrates, phosphates, hydrogen chloride, or mixtures thereof. The selection of a suitable buffer is deemed to be within the scope of those skilled in the art from the teachings herein.

The buffer is employed in a concentration which provides the desired eluting, without destroying the binding ability of the binder. In some cases, the eluting ability is enhanced by increasing the salt concentration (ionic strength) of the solution. Such increases in salt concentration may be effected by increasing the concentration of the buffer or by the addition of a water soluble salt which does not adversely affect the binder. As representative examples of such salts, there may be mentioned: water soluble salts of an alkali metal or ammonium, such as halides; sulfates, nitrates, phosphates, carbonates, bicarbonates, or water soluble transition metal salts, such as a nitrate, halide. As hereinabove noted, the total salt concentration, including buffer, is one which provides the desired eluting without destroying the binder, and in general, the salt concentration does not exceed 4M, and in most cases does not exceed 2M. In general, the salt concentration is at least 0.01M, when such salt is employed.

The present invention is concerned with an assay wherein the supported binder is regenerated, and is particularly suitable for use in an automated assay. In such an automated assay, the supported binder is provided in a flow through chamber, with the automated apparatus being, for example, of a type disclosed in U.S. Patent No. 4,009,005.

In such an assay, the sample containing or suspected of containing the antigen is incubated with the labeled antibody, and the resulting mixture is caused to flow through a chamber containing the supported binder to separate the labeled antibody bound to the antigen, from the free labeled antibody. The amount of bound and/or free labeled antibody is determined, followed by passage of an eluting liquid through the chamber to elute the antigen-labeled antibody complex from the supported antibody, whereby the chamber may be reused in an assay.

In accordance with the preferred embodiment, the antibody is labeled with a gamma emitting radioisotope and the outlet from the chamber is connected to a gamma detector, with the free labeled antibody being initially washed through the chamber and counted, followed by elution of the complex and counting of the bound labeled antibody in the detector. The percent of the total radioactivity in the bound fraction is then directly proportional to the concentration of the antigen in the standard or sample.

The invention can be performed using a suitable reagent kit or package for accomplishing such reusable assay, which includes as principal components: (a) a labeled binder for the antigen having multiple binding sites, which binder is preferably an antibody, with such antibody preferably being labeled with a radioisotope; in particular, radioiodine; (b) a binder for the antigen-labeled binder complex, which binder is supported on a solid support, with the binder preferably being an antibody and preferably being supported in a flow through chamber; and (c) an eluting liquid capable of eluting the labeled binder-antigen complex from the supported binder, with the eluting liquid generally being an aqueous eluting liquid of the type hereinabove described. Such components, where applicable are included in the reagent kit or package in separate containers; e.g., vials. The reagent kit or package may also include other components such as standards of the antigen to be assayed i.e., antigen samples having known concentrations of the antigen to be assayed; buffers, wash liquids, etc.

As hereinabove noted, the present invention has particular applicability to a radioassay for hTSH, wherein the sample containing or suspected of containing the hTSH, is mixed with a limited amount of $^{125}$Iodine labeled antibody to hTSH followed by incubation thereof. The

concentration of the [125]Iodine labeled antibody to hTSH is sufficient to convert the hTSH to a radioactive complex, and provide a free fraction of such labeled antibody; however, the labeled antibody does not completely saturate all of the binding sites of the hTSH to thereby permit subsequent binding thereof to the supported antibody.

The incubated mixture then flows through the solid support to bind the complex, and the free or unbound labeled antibody is washed therethrough into a gamma counter for counting, followed by elution of the complex, and counting of the bound antibody. In accordance with the preferred embodiment, the chamber includes antibody supported on crosslinked agarose (Sepharose)® activated with cyanogen bromide.

The invention will be further described with respect to the following examples; however, the scope of the invention is not to be limited thereby:

Example

The following reagents in appropriate containers, such as vials, are employed for a resuable assay for hTSH in accordance with the invention:

Reagents

1. hTSH antibody chamber, containing hTSH antibody covalently bound to cyanogen bromide activated crosslinked agarose.
2. hTSH prefilter, containing filtering material.
3. hTSH standards, as follows:

| Standard | hTSH Concentration in μIU/ml |
|---|---|
| Zero | 0 |
| A | 2.5 |
| B | 5 |
| C | 10 |
| D | 25 |
| E | 50 |
| F | 100 |

4. Adsorption buffer aqueous solution containing TRIS, bovine serum albumin, sodium chloride and preservative, pH. 8.0±0.1.
5. Elution buffer aqueous glycinate buffer containing preservative, pH 2.0±0.1.
6. Rinse solution of stabilized distilled water.
7. [125]I-hTSH antibody, contains <(3.7 $10^{-15}$ s$^1$ 10 μCi) on date of calibration.

The reagents may be employed in automated apparatus, as described, for example in U.S Patent No. 4,009,005, with such apparatus being available from Becton Dickinson Immuno- diagnostics, Automated Immunochemistry Systems. Such equipment is identified by the Trademark Aria II.

In employing such assay, the standard or serum hTSH sample is incubated with [125]I-hTSH antibody, for example, at 20—25°C for two hours, and the mixture is caused to flow through the antibody chamber, with the free labeled antibody being rinsed from the chamber by the rinse solution and counted in a gamma counter. The complex is then eluted from the antibody chamber by use of the elution buffer, with the eluted material flowing to the gamma counter for counting thereof. Adsorption buffer is then passed through the chamber to remove eluting buffer, whereby the chamber can be reused in the assay.

Using the above procedure for hTSH, the intra-assay reproducibility for human serum had a coefficient of variation in the order of 6—7% for samples in the normal range. Samples in the hypothyroid range also have a coefficient of variation in the order of 6—7%. Such values indicate that the assay is suitable for clinical determinations.

Although the invention has been particularly described with respect to an assay for hTSH, the teachings of the invention are also applicable to other antigens having multiple binding sites.

The assay for hTSH may be employed to determine elevated serum hTSH concentration, which result from primary hypothyroidism. There is an indication that elevation in serum hTSH levels may be the first measurable change that occurs when thyroid secretion declines.

The present invention is particularly advantageous in that it permits for effective determination of antigens having multiple binding sites. In addition, by use of the present invention, it is possible to accomplish such assay with regeneration of supported binder. Moreover, it is also possible to effect automation of such assay.

These and other advantages of the invention should be apparent to those skilled in the art from teachings herein.

Numerous modifications and variations of the present invention are possible in light of the above teachings and, therefore the invention may be practised otherwise than as particularly described.

Claims

1. A process for the assay of an antigen having multiple binding sites, the assay comprising:

contacting a sample containing said antigen with a labeled binder for said antigen to bind a portion of the labeled binder to the antigen and thereby give a mixture containing free labeled binder and a complex of labeled binder bound to said antigen; binding the said complex to a supported binder for the complex and thereby separating the bound labeled binder from the free labeled binder; and determining the amount of at least one of the bound and the free labeled binder;

characterized in that the process is carried out using a flow-through chamber containing the supported binder for the complex, whereby the mixture is flowed through the chamber to bind the complex to the supported binder and an eluting liquid is then flowed through the chamber to elute the complex from the supported binder.

2. A process according to claim 1, wherein the labeled binder is radiolabeled.

3. A process according to claim 2, wherein the labeled binder is labeled with $^{125}$I.

4. A process according to claim 1, 2 or 3, wherein the labeled binder and the supported binder are each antibodies.

5. A process according to claim 4, wherein the antigen is a polypeptide or protein.

6. A process according to claim 5, wherein the antigen is hTSH.

7. A process according to any preceding claim, wherein the labeled binder is employed in an amount in excess of that required to bind all of the antigen binding sites for the labeled binder, with said antigen having remaining binding sites.

8. A process according to any preceding claim, wherein the complex is eluted from the supported binder by an aqueous eluting liquid buffered to a pH of no greater than 3.0 and no less than 1.5 to permit reuse of the supported binder.

9. A process according to claim 8, wherein the eluting liquid is buffered to a pH of no greater than 2.0.

10. A process according to any preceding claim, wherein the supported binder is supported on cyanogen bromide-activated crosslinked agarose.

## Patentansprüche

1. Verfahren für den Assay eines Antigens mit Mehrfach-Bindungsstellen, wobei der Assay umfaßt: Kontaktieren einer das genannte Antigen enthaltenden Probe mit einem markierten Bindungspartner für das genannte Antigen, um einen Teil des markierten Bindungspartners an das Antigen zu binden und dadurch eine Mischung zu ergeben, die freien markierten Bindungspartner und einen Komplex von an das genannte Antigen gebundenem Bindungspartner enthält Binden des genannten Komplexes an einen trägerfixierten Bindungspartner für den Komplex und dadurch Trennen des gebundenen markierten Bindungspartners von dem freien markierten Bindungspartner und Bestimmen der Menge wenigstens eines der beiden, d.h. des gebundenen bzw. des freien markierten Bindungspartners, dadurch gekennzeichnet, daß das Verfahren unter Verwendung einer Durchfluß-Kammer durchgeführt wird, die den trägerfixierten Bindungspartner für den Komplex enthält, daß die Mischung durch die Kammer fließen gelassen wird, un den Komplex an den trägerfixierten Bindungspartner zu binden, und daß dann eine Eluier-Flüssigkeit durch die Kammer fließen gelassen wird, un den Komplex von dem trägerfixierten Bindungspartner auszuwaschen.

2. Verfahren nach Anspruch 1, dadurch gekennzeichnet, daß der markierte Bindungspartner radiomarkiert ist.

3. Verfahren nach Anspruch 2, dadurch gekennzeichnet, daß der markierte Bindungspartner mit $^{125}$I markiert ist.

4. Verfahren nach Anspruch 1, 2 oder 3, dadurch gekennzeichnet, daß der markierte Bindungspartner und der trägerfixierte Bindungspartner jeweils Antikörper sind.

5. Verfahren nach Anspruch 4, dadurch gekennzeichnet, daß das Antigen ein Polypeptid oder Protein ist.

6. Verfahren nach Anspruch 5, dadurch gekennzeichnet, daß das Antigen hTSH ist.

7. Verfahren nach einem der voraufgehenden Ansprüche, dadurch gekennzeichnet, daß der markierte Bindungspartner in einer Menge im Überschuß über diejenige verwendet wird, die erforderlich ist, um alle Antigen-Bindungsstellen für den markierten Bindungspartner zu binden, wobei das genannte Antigen verbleibende Bindungsstellen hat.

8. Verfahren nach einem der voraufgehenden Ansprüche, dadurch gekennzeichnet, daß der Komplex von dem trägerfixierten Bindungspartner mittels einer wässrigen Eluier-Flüssigkeit ausgewaschen wird, die auf einen pH-Wert nicht größer als 3,0 und nicht kleiner als 1,5 gepuffert ist, um eine Wiederverwendung des trägerfixierten Bindungspartners zu gestatten.

9. Verfahren nach Anspruch 8, dadurch gekennzeichnet, daß die Eluier-Flüssigkeit auf einen pH-Wert nicht größer als 2,0 gepuffert ist.

10. Verfahren nach einem der voraufgehenden Ansprüche, dadurch gekennzeichnet, daß der trägerfixierte Bindungspartner an Zyanbromidaktivierter vernetzter Agarose vorgesehen ist.

## Revendications

1. Procédé de dosage d'un antigène comportant de multiples sites de liaison, le dosage comprenant:

la mise d'un échantillon, contenant ledit antigène, en contact avec un liant, marqué, de cet antigène pour lier à l'antigène une partie du liant marqué et obtenir ainsi un mélange contenant du liant marqué libre et un complexe liant marqué lié audit antigène; la liaison dudit complexe à un liant, sur support, du complexe et ainsi la séparation du liant marqué et lié d'avec le liant marqué libre; et la détermination de la quantité d'au moins l'un des liants marqués, celui qui est lié ou celui qui est libre:

procédé caractérisé en ce qu'on le met en oeuvre en utilisant une chambre à écoulement continu contenant le liant, sur support, du complexe, en ce qu'on fait couler le mélange dans la chambre pour lier le complexe au liant sur son support et l'on fait ensuite couler dans la chambre un liquide d'élution pour éluer le complexe du liant sur son support.

2. Procédé selon la revendication 1, caractérisé en ce que liant marqué est marqué par un radioisotope.

3. Procédé selon la revendication 2, caractérisé en ce que la liant marqué est marqué par $^{125}$I.

4. Procédé selon la revendication 1, 2 ou 3, dans lequel le liant marqué et le liant sur sont support sont chacun des anticorps.

5. Procédé selon la revendication 4, dans lequel l'antigène est un polypeptide ou une protéine.

6. Procédé selon la revendication 5, dans lequel l'antigène est hTSH.

7. Procédé selon l'une quelconque des revendications précédentes, dans lequel on utilise le liant marqué en une quantité excédant celle nécessaire pour lier la totalité des sites de liaison de l'antigène au liant marqué, cet antigène comportant des sites restants de liaison.

8. Procédé selon l'une quelconque des revendications précédentes, dans lequel on élue le complexe du liant sur son support à l'aide d'un liquid aqueux d'élution tamponné à un pH non supérieur à 3,0 et non inférieur à 1,5, pour permettre une réutilisation du liant sur son support.

9. Procédé selon la revendication 8, dans lequel le liquide d'élution est tamponné à un pH non supérieur à 2,0.

10. Procédé selon l'une quelconque des revendications précédentes, dans lequel le liant sur support est placé sur un support de gélose réticulée, activée par du bromure de cyanogène.